(19) **Europäisches Patentamt / European Patent Office / Office européen des brevets**

(11) **EP 4 145 452 A1**

(12) # EUROPEAN PATENT APPLICATION
published in accordance with Art. 153(4) EPC

(43) Date of publication:
**08.03.2023 Bulletin 2023/10**

(21) Application number: **21795631.7**

(22) Date of filing: **09.04.2021**

(51) International Patent Classification (IPC):
**G16C 20/40** (2019.01)

(52) Cooperative Patent Classification (CPC):
**G06F 16/9032; G16C 20/40**

(86) International application number:
**PCT/JP2021/015045**

(87) International publication number:
**WO 2021/220777 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2020 JP 2020080755**

(71) Applicant: **Hitachi, Ltd.**
**Tokyo 100-8280 (JP)**

(72) Inventors:
• **ASAHARA, Akinori**
  **Tokyo 100-8280 (JP)**
• **MORITA, Hidekazu**
  **Tokyo 100-8280 (JP)**
• **HAYASHI ,Takayuki**
  **Tokyo 100-8280 (JP)**
• **KANAZAWA ,Takuya**
  **Tokyo 100-8280 (JP)**
• **OSAKABE, Yoshihiro**
  **Tokyo 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(54) **SYSTEM FOR DETERMINING MATERIAL TO BE PROPOSED TO USER**

(57)      A system for determining a material to propose to a user is disclosed. The system calculates an availability evaluation value indicating availability for a user of each of materials based on a chemical formula of each of the materials. The system estimates a physical property value of each of the materials based on a chemical formula of each of the materials. The system calculates a physical property evaluation value of each of the materials based on an estimation result of the physical property value of each of the materials. The system calculates an overlooking risk evaluation value indicating priority of presenting each of the materials to the user based on the availability evaluation value and the physical property evaluation value of each of the materials. The system selects a material to present as a candidate material from the materials according to the overlooking risk evaluation value.

*FIG. 6*

**Description**

Incorporation by Reference

This application claims priority to Japanese Patent Application No. 2020 080755 filed on April 30, 2020, the contents of which are incorporated herein by reference.

Technical Field

The present invention relates to a system for determining a material to be proposed to a user.

Background Art

A virtual screening method is used for a new material searching task. In the virtual screening, a machine learning model is applied to data of a known compound, and a physical property value estimation model having a descriptor of the compound as an input is configured. The physical property value estimation model is applied to the newly generated chemical formula, and screening is performed based on the calculated estimated value. As a result, a new compound candidate that matches the purpose of the user can be presented.

Another related art of the present specification is Japanese Patent Laid-open Publication No. 2014-92930. This document discloses that "the information providing device 101 acquires a compound name of a target compound. The information providing device 101 searches the database 110 for a chemical formula candidate corresponding to the acquired compound name of the target compound. The information providing device 101 extracts a character string representing a substituent of the target compound from the compound name of the target compound. The information providing device 101 specifies a bonding position of a substituent bonded to a mother nucleus of a compound having a compound name including a character string representing an extracted substituent among a group of compound names stored in the database 110 in association with chemical formula candidates, thereby calculating the number of variations of the bonding position of the substituent. The information providing device 101 outputs the calculated number of variations of bonding position of the substituent in association with the chemical formula candidate." (Abstract).

Citation List

Patent Literature

PTL 1: JP 2014-92930 A

Summary of Invention

Technical Problem

In a case where compound candidates presented only by the physical property value estimation result are selected in the virtual screening, there is a case where they are unavailable to the user. The compound unavailable to the user is, for example, a compound that cannot be synthesized or is not practical to the user from the standpoint of cost, yield, stability, or the like. Such compounds should be excluded from the candidates of the compound to be presented. On the other hand, a compound similar to an unavailable compound may have an extremely excellent physical property value.

JP 2014 -92930 A discloses that a candidate of a structural formula is created from a compound name and presented to a user, but does not disclose presenting a new compound desirable to the user. Therefore, a technology capable of more appropriately presenting a compound candidate desired by the user is desired.

Solution to Problem

One aspect of the present invention is a system for determining a material to be proposed to a user, the system including one or more processors; and one or more storage devices that store a program executed by the one or more processors. The one or more processors calculate an availability evaluation value indicating availability for a user of each of the plurality of materials based on a chemical formula of each of the plurality of materials. The one or more processors estimate a physical property value of each of the plurality of materials based on a chemical formula of each of the plurality of materials. The one or more processors calculate a physical property evaluation value of each of the plurality of materials based on an estimation result of the physical property value of each of the plurality of materials.

The one or more processors calculate an overlooking risk evaluation value indicating priority of presenting each of the plurality of materials to the user based on the availability evaluation value and the physical property evaluation value of each of the plurality of materials. The one or more processors select a material to be presented as a candidate material from the plurality of materials according to the overlooking risk evaluation value.

Advantageous Effects of Invention

According to one aspect of the present invention, a compound candidate desired by a user can be more appropriately presented.

Brief Description of Drawings

[FIG. 1] FIG. 1 illustrates an example of a configuration of an experimental design support system that supports an experimental design of a compound (material) to be newly synthesized according to a first embodiment.
[FIG. 2] FIG. 2 illustrates a hardware configuration example of an experimental design support device and a client computer.
[FIG. 3] FIG. 3 illustrates a configuration example of an experimental database.
[FIG. 4] FIG. 4 illustrates a configuration example of an availability database.
[FIG. 5] FIG. 5 illustrates a process of presenting an experimental candidate compound for the first time.
[FIG. 6] FIG. 6 illustrates second and subsequent experimental candidate compound presenting processes.
[FIG. 7] FIG. 7 illustrates a flowchart of an example of a first experimental candidate compound presenting process.
[FIG. 8] FIG. 8 illustrates an example of a candidate presentation request input screen displayed on a monitor of a client computer by a candidate compound display unit.
[FIG. 9] FIG. 9 illustrates a configuration example of a candidate presentation request.
[FIG. 10] FIG. 10 schematically illustrates processes of generation of a candidate compound, estimation of physical property values of the candidate compound, and selection of a compound to be presented from the candidate compound.
[FIG. 11] FIG. 11 illustrates a flowchart of an example of second and subsequent experimental candidate compound presenting processes.
[FIG. 12] FIG. 12 illustrates an example of a candidate addition request input screen displayed on the monitor of the client computer by the candidate compound display unit.
[FIG. 13] FIG. 13 illustrates a configuration example of a candidate addition request.
[FIG. 14] FIG. 14 is a flowchart of an example of a candidate compound selecting process based on user feedback.
[FIG. 15] FIG. 15 illustrates a configuration example of a candidate addition request according to a second embodiment.
[FIG. 16] FIG. 16 illustrates a configuration example of an availability database according to the second embodiment.
[FIG. 17] FIG. 17 is a flowchart of an example of a candidate compound selecting process based on user feedback according to the second embodiment.

Description of Embodiments

In the following description, when it is necessary for the sake of convenience, the description will be divided into a plurality of sections or embodiments, but unless otherwise specified, the sections or examples are not unrelated to each other, and one is in a relationship of some or all modified examples, details, supplementary explanations, and the like of the other. Furthermore, in the following, when referring to the number of elements and the like (including number, numerical value, amount, range, etc.), the number of elements is not limited to a specific number unless otherwise stated or unless clearly limited to the specific number in principle, and the number of elements may be greater than or equal to or less than or equal to the specific number.

The present system may be a physical computer system (one or more physical computers) or a system built on a calculation resource group (a plurality of calculation resources) such as a cloud foundation. The computer system or the calculation resource group includes one or more interface devices (e.g., including a communication device and an input/output device), one or more storage devices (e.g., including a memory (main storage) and an auxiliary storage device), and one or more processors.

In a case where the function is realized by executing the program by the processor, the determined process is appropriately performed using the storage device and/or the interface device, and thus the function may be at least a part of the processor. The process described with the function as the subject may be a process performed by a processor or a system including the processor. The program may be installed from a program source. The program source may be, for example, a program distribution computer or a computer-readable storage medium (e.g., a computer-readable non-transitory storage medium). The description of each function is an example, and a plurality of functions may be integrated into one function or one function may be divided into a plurality of functions.

[Outline]

Hereinafter, a system that supports development of a new compound by a user is disclosed. The system according to an embodiment of the present specification presents materials to be candidates for experiments and simulations to a user. The system quantifies the evaluation of the physical properties of the candidate material and the availability of the candidate material and calculates an evaluation value of the candidate material (hereinafter also referred to as overlooking evaluation value) based thereon. The system ranks the candidate materials according to their evaluation values and presents the high-ranking candidate materials to the user.

In this way, by quantifying the evaluation of each of the physical properties and the availability of the candidate material and selecting the candidate material to be presented to the user by the evaluation value obtained by combining the above, the possibility of presenting a material that is not available to the user can be reduced and the possibility of presenting a material having excellent physical properties even though the availability is low can be increased. Therefore, it is possible to efficiently search for a new material having desired physical properties by the user.

First Embodiment

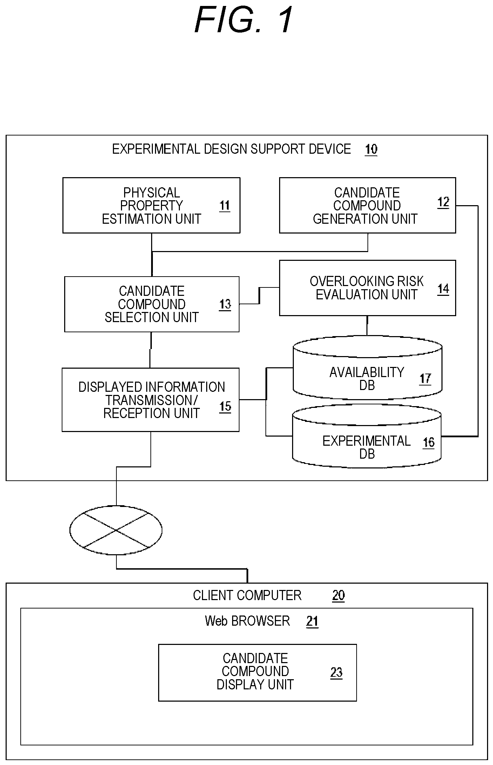

FIG. 1 illustrates an example of a configuration of an experimental design support system that supports an experimental design of a compound (material) to be newly synthesized according to a first embodiment. Note that the user may design a simulation in addition to or instead of the compound experiment with the support of the present system. The system includes an experimental design support device 10 and a client computer 20 that can communicate with each other via a network.

The experimental design support device 10 presents candidates of a compound (material) for which the user newly conducts an experiment or simulation. Hereinafter, for ease of explanation, a candidate compound presented by the experimental design support device 10 is assumed as an experimental target candidate to be newly synthesized. In addition to displaying the candidate compound presented by the experimental design support device 10 to the user, the client computer 20 transmits feedback on the presented compound input from the user to the experimental design support device 10.

In the configuration example of FIG. 1, the experimental design support device 10 includes a physical property estimation unit 11, a candidate compound generation unit 12, a candidate compound selection unit 13, an overlooking risk evaluation unit 14, and a displayed information transmission/reception unit 15. These are programs. The experimental design support device 10 further stores an experimental database (DB) 16 and an availability database 17.

The client computer 20 stores and executes a Web browser 21 which is a program. The Web browser 21 includes a candidate compound display unit 23. This is an internal module of the Web browser 21.

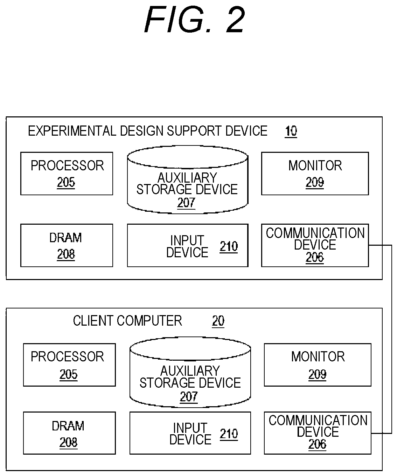

FIG. 2 illustrates a hardware configuration example of the experimental design support device 10 and the client computer 20. In the configuration example of FIG. 2, the experimental design support device 10 includes a processor 205 having calculation performance and a DRAM 208 that provides a volatile temporary storage area for storing programs executed by the processor 205 and data.

The experimental design support device 10 further includes a communication device 206 that performs data communication with other devices including the client computer 20, and an auxiliary storage device 207 that provides a permanent information storage area using a hard disk drive (HDD), a flash memory, or the like.

For example, the auxiliary storage device 207, the experimental design support device 10 stores programs of the physical property estimation unit 11, the candidate compound generation unit 12, the candidate compound selection unit 13, the overlooking risk evaluation unit 14, the displayed information transmission/reception unit 15, and the like. The auxiliary storage device 207 further stores various data such as the experimental database 16 and the availability database 17. The programs executed by the processor 205 and the data to be processed are loaded from the auxiliary storage device 207 to the DRAM 208.

Furthermore, the experimental design support device 10 includes an input device 210 that receives an operation from the user, and a monitor 209 (an example of an output device) that presents an output result in each process to the user.

The client computer 20 includes hardware components similar to those of the experimental design support device 10, and the same components are denoted with the same reference numerals. Note that functions divided to a plurality of devices may be integrated in one device, or the plurality of functions may be distributed to more devices. In addition, distributed functions of a plurality of devices may be integrated in one device. As described above, the experimental design support device 10, the client computer 20, and the system including them include one or more storage devices and one or more processors.

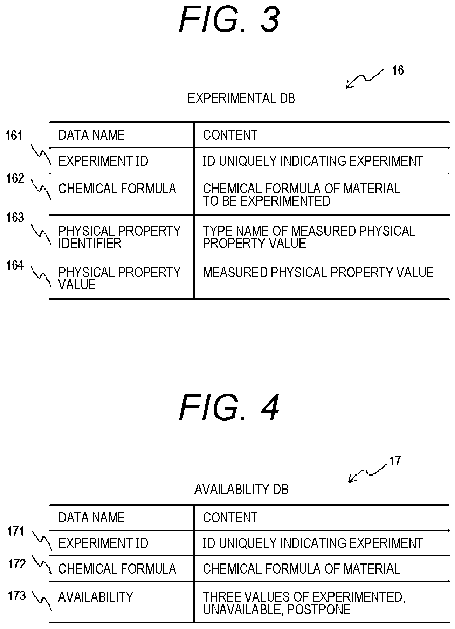

FIG. 3 illustrates a configuration example of an experimental database 16. The experimental database 16 stores information of the experiment results of the compound, and stores data of the material for which physical property values of interest have been measured. Each record stored in the experimental database 16 includes fields for an experiment ID 161, a chemical formula 162, a physical property identifier 163, and a physical property value 164.

The experiment ID 161 indicates an ID uniquely indicating the experiment. A plurality of records include the same experiment ID 161. As will be described later, the experimental design support device 10 presents candidates of a compound to be newly synthesized and experimented with respect to experimental data (record group) identified by the experiment ID. The chemical formula 162 indicates a chemical formula of the material to be experimented. The chemical formula includes a chemical structural formula and a composition formula, and an appropriate chemical formula is assigned according to the material.

The physical property identifier 163 indicates an identifier of the measured physical property value, for example, a type name of the physical property value. As the type of physical property value, both a type representing a physical property and a type representing a chemical property can be adopted. The physical property value 164 indicates a measured physical property value. Note that the experimental database 16 may include information on simulation result in addition to or in place of the experimental data. The physical property value 164 indicates a measurement value by simulation instead of a measurement value by experiment.

FIG. 4 illustrates a configuration example of an availability database 17. The availability database 17 stores information on availability for the user of each of the plurality of materials. As will be described later, the availability database 17 stores information on feedback from the user with respect to the candidate compound presented to the user. The feedback includes information on the availability of the candidate compound.

In the example of FIG. 4, the records in the availability database 17 include fields for experiment ID 171, chemical formula 172, and availability 173. The experiment ID 171 indicates an ID uniquely indicating an experiment, and is common to the experiment ID 161 of the experimental database 16. The chemical formula 172 indicates a chemical formula of the target material. The availability 173 indicates whether the target material is available to the user.

The compound unavailable to the user is a compound that the user does not use, and is, for example, a compound that cannot be synthesized or is not practical to the user from the standpoint of cost, yield, stability, or the like. The available materials are materials that a user may possibly use. Thus, the availability/unavailability of a material in the availability database 17 is designated by the user.

In the examples described below, the availability 173 of each record indicates one of "experimented", "unavailable", or "postpone". "Experimented" indicates that the experiment of the material has been executed and is available to the user. "Unavailable" indicates that it is unavailable by the user. "Postpone" indicates that it is available to the user, but the experiment has been postponed. Note that these are examples, and the availability 173 can define an arbitrary category that indicates whether or not the material is available to the user. For example, "postpone" may be omitted and available material and unavailable material may be indicated regardless of the presence or absence of experiment.

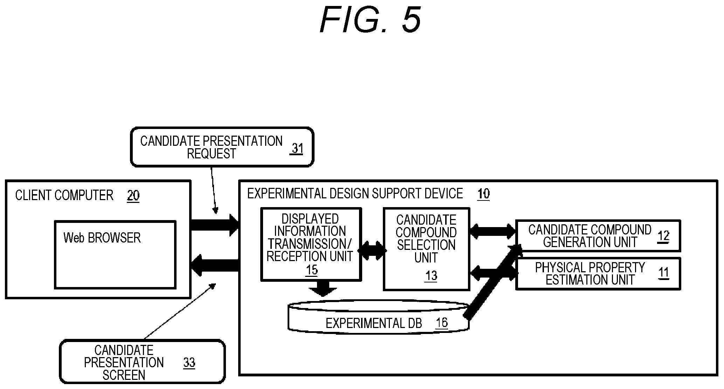

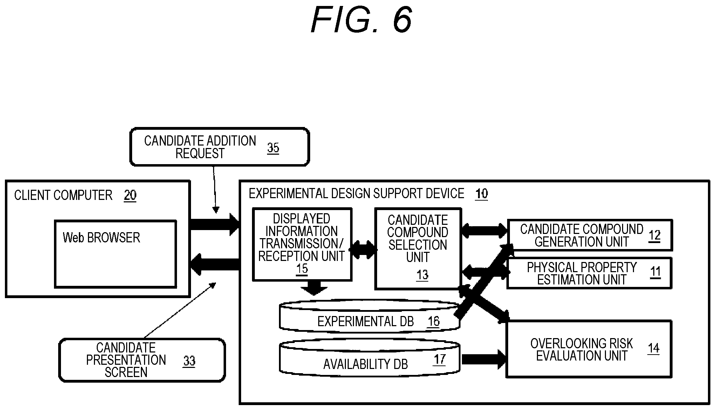

FIGS. 5 and 6 schematically illustrate an outline of processing of the experimental design support system. FIG. 5 illustrates a process of presenting an experimental candidate compound for the first time, and FIG. 6 illustrates a second and subsequent experimental candidate compound presenting processes. The user conducts an experiment of a compound selected from the candidate compounds presented by the experimental design support system, and feeds back the experiment result to the experimental design support system.

The experimental design support system receives the feedback and presents the new candidate compound. The experimental design support system and the user repeat presentation of the candidate compound and the experiment. A new material search including these series of presentation and experiments is also referred to as a project. The same presentation process ID is assigned to the series of candidate presentation, and associated with the experiment ID.

Referring to FIG. 5, the user operates the client computer 20 with the input device 210 while referring to the screen of the monitor 209. The user transmits a candidate presentation request 31 to the experimental design support device 10 through the candidate compound display unit 23 of the Web browser 21.

In response to the received candidate presentation request 31, the displayed information transmission/reception unit 15 of the experimental design support device 10 requests the candidate compound selection unit 13 to select a candidate compound to present to the user. The candidate compound selection unit 13 requests the candidate compound generation unit 12 to generate a candidate compound list. The candidate compound generation unit 12 generates a candidate compound list indicating the candidate compounds.

The candidate compound generation unit 12 generates a new compound from the compound of the experimental data corresponding to the candidate presentation request 31 in the experimental database 16. In the embodiment of the present specification, the experimental database 16 stores in advance initial data corresponding to the candidate presentation request 31. The candidate compound generation unit 12 may use data of a compound different from the experimental data (e.g., open data or initial data set in advance in the system).

The candidate compound selection unit 13 receives the candidate compound list from the candidate compound generation unit 12. Furthermore, the candidate compound selection unit 13 requests the physical property estimation unit 11 to estimate the physical property value of each of the candidate compounds indicated by the candidate compound list. The physical property estimation unit 11 estimates a physical property value of each of the compounds listed in the candidate compound list through a method defined in advance.

As will be described later, for example, one physical property value may be estimated, or a probability distribution of the physical property values may be estimated. The estimated probability distribution also corresponds to the estimated physical property value. The number of physical property value types to be estimated may be one or more. Hereinafter, an example in which the number of physical property value types to be estimated is one will be specifically described.

The candidate compound selection unit 13 receives physical property values of each of the compounds listed in the candidate compound list from the physical property estimation unit 11. The candidate compound selection unit 13 selects a candidate compound to present to the user from the candidate compound list based on the estimation result of the physical property value without considering the availability.

The candidate compound selection unit 13 passes information on each of the selected candidate compounds to the displayed information transmission/reception unit 15. The displayed information transmission/reception unit 15 generates (data of) a candidate presentation screen 35 indicating information on each of the selected candidate compounds, and transmits the candidate presentation screen 33 to the client computer 20.

The user executes the Web browser 21 on the client computer 20, and the candidate compound display unit 23 displays the candidate presentation screen 33 on the monitor 209. The user refers to the candidate presentation screen 33 to select a compound to be subjected to an experiment from among the candidate compounds. The user conducts experiments on the selected compounds and measures their physical property values.

Next, second and subsequent experimental candidate compound presenting processes will be described with reference to FIG. 6. After conducting an experiment of a compound selected from the previously presented candidate compounds, the user transmits the feedback thereof to the experimental design support device 10. Specifically, the user operates the client computer 20 with the input device 210 while referring to the screen of the monitor 209. The user transmits a candidate addition request 35 to the experimental design support device 10 through the candidate compound display unit 23 of the Web browser 21. The candidate addition request 35 includes feedback information from the user with respect to a previous presentation candidate compound including experiment results.

The displayed information transmission/reception unit 15 of the experimental design support device 10 stores the feedback information included in the candidate addition request 35 in the experimental database 16 and the availability database 17. The feedback information in the candidate addition request 35 includes information of the experiment result and information of availability designated by the user with respect to the previous presentation candidate compound.

Next, the displayed information transmission/reception unit 15 requests the candidate compound selection unit 13 to select a candidate compound to be additionally presented to the user. The candidate compound selection unit 13 requests the candidate compound generation unit 12 to generate a candidate compound list. The candidate compound generation unit 12 generates a candidate compound list indicating the candidate compounds. The candidate compound generation unit 12 generates, for example, a new compound from the compounds of the data of the experiment ID of the candidate addition request 35 in the experimental database 16.

The candidate compound selection unit 13 receives the candidate compound list from the candidate compound generation unit 12. Furthermore, the candidate compound selection unit 13 requests the physical property estimation unit 11 to estimate the physical property value of each of the candidate compounds indicated by the candidate compound list. The physical property estimation unit 11 estimates a physical property value of each of the compounds listed in the candidate compound list through a method defined in advance. The estimation of the physical property value is similar to the estimation of the physical property value in the first candidate compound presentation. The estimation method may be different between the first presentation and the second and subsequent presentations.

The candidate compound selection unit 13 receives physical property values of each of the compounds listed in the candidate compound list from the physical property estimation unit 11. The candidate compound selection unit 13 further requests the overlooking risk evaluation unit 14 to evaluate the overlooking risk. The overlooking risk evaluation unit 14 calculates an overlooking risk evaluation value of each of the candidate compounds based on the estimated physical property value and the evaluation value of each availability of the candidate compound on which the candidate compound list is formed. The overlooking risk evaluation value indicates the priority to be presented to the user.

For example, the overlooking risk evaluation unit 14 evaluates the availability of the candidate compound based on the information in the availability database 17. The availability for the user can be more appropriately calculated by calculating the availability evaluation value based on the information of the availability designated by the user. In another example, the overlooking risk evaluation unit 14 may evaluate the availability by an evaluation value (e.g., SA score) indicating the synthesis difficulty of the candidate compound. In this case, the availability database 17 can be omitted.

The candidate compound selection unit 13 acquires an overlooking risk evaluation value of each of the candidate compounds from the overlooking risk evaluation unit 14. The candidate compound selection unit 13 ranks the candidate compounds based on the overlooking risk evaluation value, and selects a candidate compound to present to the user from the candidate compound list. The candidate compound selection unit 13 passes information on each of the selected candidate compounds to the displayed information transmission/reception unit 15.

The displayed information transmission/reception unit 15 generates (data of) a candidate presentation screen 35 indicating information on each of the selected candidate compounds, and transmits the candidate presentation screen 33 to the client computer 20. The user executes the Web browser 21 on the client computer 20, and the candidate compound display unit 23 displays the candidate presentation screen 33 on the monitor 209. The user refers to the candidate presentation screen 33 to select a compound to be subjected to an experiment from among the candidate compounds. The user conducts experiments on the selected compounds and measures their physical property values.

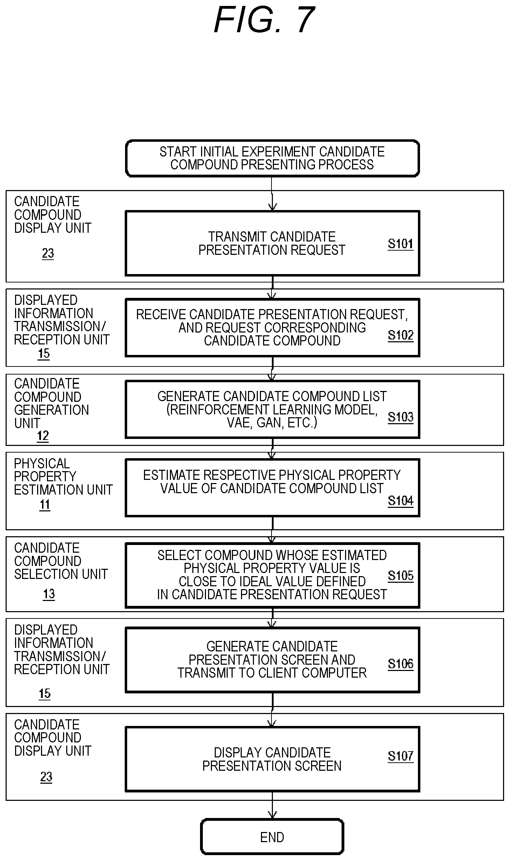

Hereinafter, details of the experimental candidate compound presenting process will be described. FIG. 7 illustrates a flowchart of an example of a first experimental candidate compound presenting process. First, in step S101, the candidate compound display unit 23 executed by the client computer 20 transmits a candidate presentation request to the experimental design support device 10. The user executes the browser 21 on the client computer 20, and inputs information of the candidate presentation request in the screen displayed by the candidate compound display unit 23.

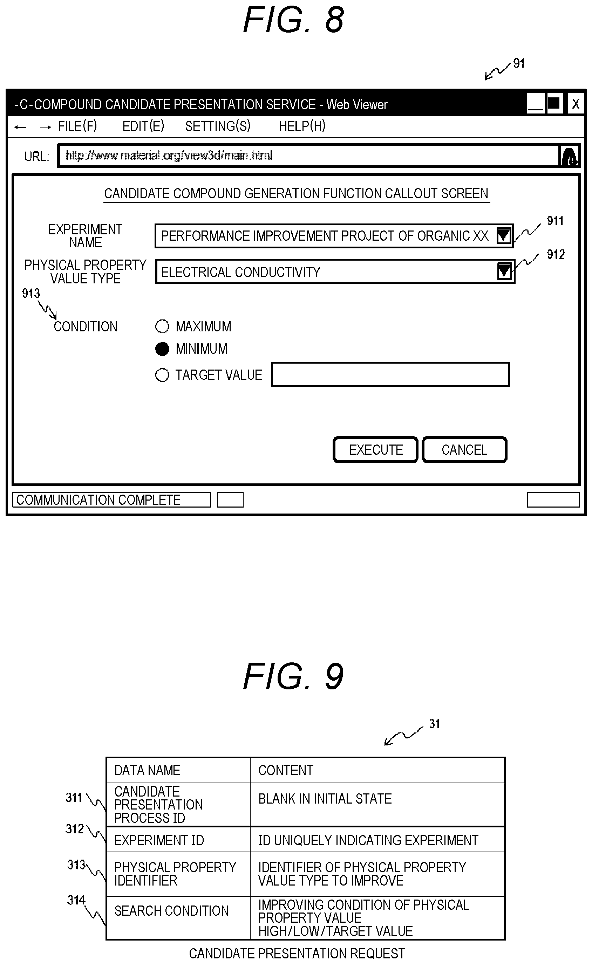

FIG. 8 illustrates an example of a candidate presentation request input screen 91 displayed on the monitor 209 of the client computer 20 by the candidate compound display unit 23. The candidate compound display unit 23 acquires the candidate presentation request input screen 91, for example, from the experimental design support device 10. The candidate presentation request input screen 91 includes an experiment name section 911, a physical property value type section 912, and a physical property value condition section 913.

The user inputs an experiment name representing the current project in the experiment name section 911. The experiment name is associated with the experiment ID. In the present example, the user selects an experiment name for which experimental data already exists from the pull-down menu. The user inputs a target physical property value type for which a request for a value exists in the physical property value type section 912. In the present example, only one physical property value type is designated, but a plurality of physical property value types may be designated.

Furthermore, the user inputs a condition required for the physical property value of the above type. In the example of FIG. 8, the condition is maximization of a physical property value, minimization of a physical property value, or a target value of a physical property value. When the target value is set, a compound estimated to have a physical property value close to the target value is presented.

The candidate compound display unit 23 generates a candidate presentation request 31 from the information input to the candidate presentation request input screen 91. FIG. 9 illustrates a configuration example of the candidate presentation request 31. The candidate presentation request 31 includes fields for a candidate presentation process ID 311, an experiment ID 312, a physical property identifier 313, and a search condition 314.

The candidate presentation process ID 311 is blank (e.g., NULL value) in the initial state, and when the experimental design support device 10 receives the candidate presentation request 31, the candidate presentation process ID 311 is assigned. The experiment ID 312 is an ID associated in advance with the experiment name 911 input by the user. The physical property identifier 313 indicates an identifier associated in advance with the input physical property value type 912. The search condition 314 indicates the input condition 913. Note that, in the present specification, the identifier and the ID are the same in that they are values for identifying a target.

Returning to FIG. 7, in step S102, the displayed information transmission/reception unit 15 receives the candidate presentation request 31, and requests the candidate compound selection unit 13 for the corresponding experimental candidate compound. The displayed information transmission/reception unit 15 assigns a new candidate presentation process ID 311 to the candidate presentation request 31. The displayed information transmission/reception unit 15 stores the candidate presentation request 31 in a database (not illustrated). The displayed information transmission/reception unit 15 requests the candidate compound selection unit 13 for an experimental candidate compound to present along with the candidate presentation request 31.

Next, in step S103, the candidate compound generation unit 12 generates a candidate compound list. Specifically, the candidate compound selection unit 13 requests the candidate compound generation unit 12 to generate a candidate compound together with the candidate presentation request 31. The candidate compound generation unit 12 generates a candidate compound list indicating the candidate compounds, and passes it to the candidate compound selection unit 13. The candidate compound generation unit 12 can generate, for example, a new candidate compound through an arbitrary method from the compounds of the experimental data indicated by the experiment ID 312 in the experimental database 16.

For example, the candidate compound generation unit 12 can generate a descriptor of a new compound from descriptors of existing compounds by a reinforcement learning model, a variational auto-encoder (VAE), a Generative Adversarial Network (GAN), or the like.

Next, in step S104, the physical property estimation unit 11 estimates the respective physical property values of the candidate compound list. Specifically, the candidate compound selection unit 13 requests the physical property estimation unit 11 to estimate the physical property value together with the candidate compound list. The physical property estimation unit 11 can estimate the physical property value of each compound in the candidate compound list from the descriptor of the compound using, for example, a machine learning model or a density functional method. For example, the physical property estimation unit 11 estimates a single physical property value or a probability distribution of the physical property values. One expected value (estimated value) can also be determined from the probability distribution.

Next, in step S105, the candidate compound selection unit 13 selects a compound whose estimated physical property value is close to the ideal value defined in the candidate presentation request. The candidate compound selection unit 13 selects a compound based on the condition designated by the candidate presentation request 31. When maximization or minimization of the physical property values is designated, the ideal value is, for example, a maximum value or a minimum value in the estimated value. When the target value is designated, the ideal value is the target value.

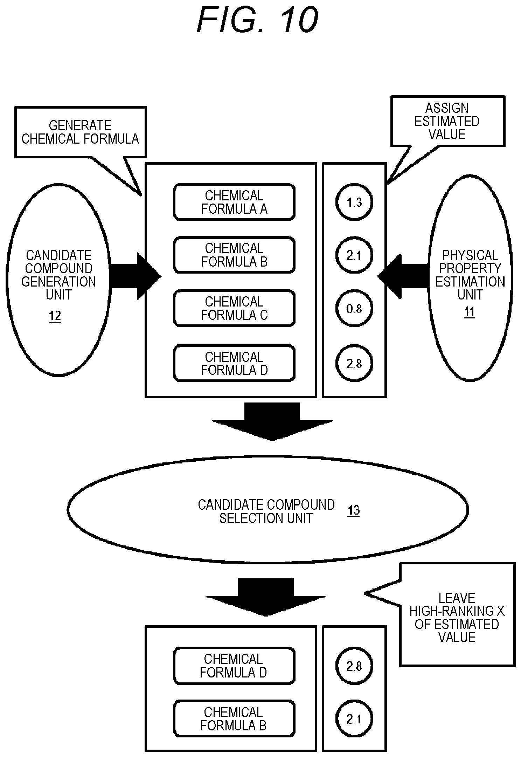

FIG. 10 schematically illustrates processes of generation of a candidate compound, estimation of physical property values of the candidate compound, and selection of a compound to be presented from the candidate compound. As described above, the candidate compound generation unit 12 generates new candidate compounds (chemical formulas A to D) from the compounds in the experimental database 16. The physical property estimation unit 11 estimates the value of the designated physical property value type of each of the candidate compounds. The candidate compound selection unit 13 selects high-ranking X compounds whose estimated physical property value is close to the ideal value. In the example of FIG. 10, two compounds (chemical formula B and chemical formula D) having the highest estimated physical property values are selected.

Returning to FIG. 7, in step S106, the displayed information transmission/reception unit 15 generates the candidate presentation screen 33 and transmits the screen to the client computer 20. The candidate presentation screen 33 includes information on the candidate compound selected by the candidate compound selection unit 13. For example, the candidate presentation screen 33 shows a chemical formula (chemical structural formula or composition formula) and an estimated physical property value of the candidate compound. The displayed information transmission/reception unit 15 stores information of the selected candidate compound in a database (not illustrated) in association with the candidate presentation request. In step S107, the candidate compound display unit 23 displays the candidate presentation screen 33 on the monitor 209.

Next, the second and subsequent experimental candidate compound presenting processes (experimental candidate compound additional presenting process) will be described. In the second and subsequent times, the experimental design support device 10 performs an experimental candidate compound selection in consideration of the feedback information from the user.

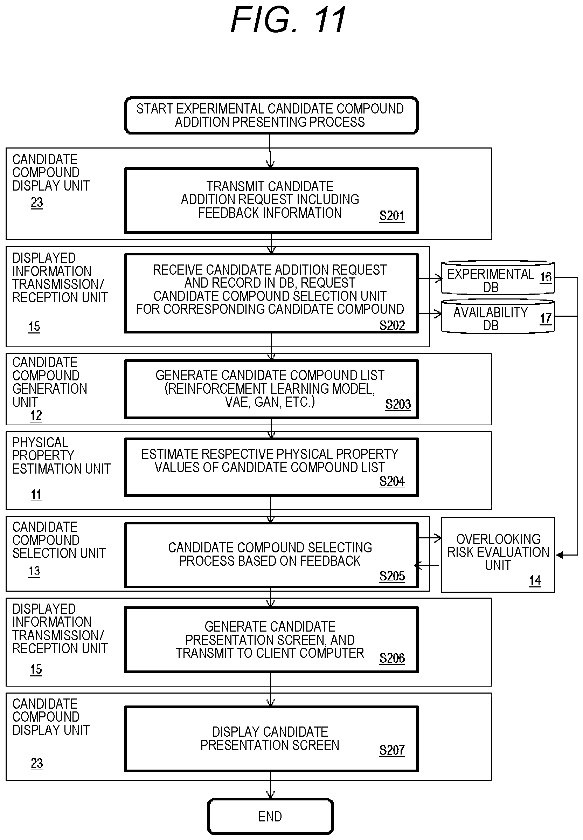

FIG. 11 illustrates a flowchart of an example of second and subsequent experimental candidate compound presenting processes. First, in step S201, the candidate compound display unit 23 executed by the client computer 20 transmits a candidate addition request 35 including the feedback information from the user to the experimental design support device 10.

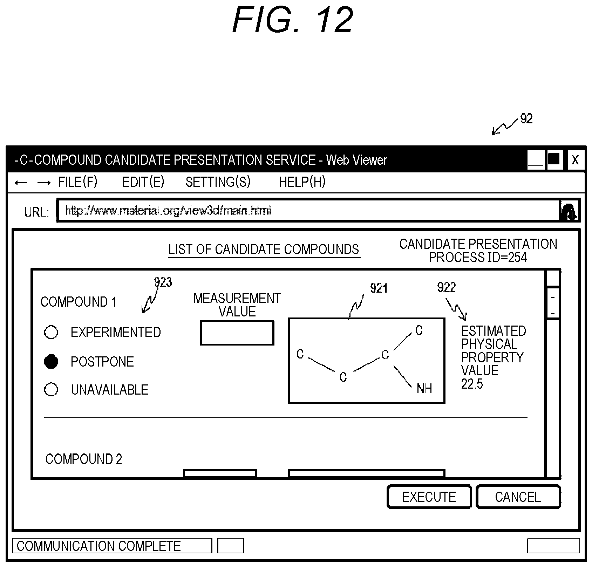

The user executes the browser 21 on the client computer 20, and inputs information of the candidate addition request in the screen displayed by the candidate compound display unit 23. FIG. 12 illustrates an example of a candidate addition request input screen 92 displayed on the monitor 209 of the client computer 20 by the candidate compound display unit 23. The candidate addition request input screen 92 shows information on the previously presented candidate compound and feedback information with respect to the candidate compound input by the user in the screen 92. The candidate addition request input screen 92 indicates a candidate presentation process ID common to candidate presentation in a series of one project.

For example, the user designates an experiment name and a candidate presentation process ID, and the candidate compound display unit 23 acquires a candidate addition request input screen 92 of the designated project from the experimental design support device 10. Note that the experimental candidate compound presentation screen may have the section of the feedback information eliminated from the candidate addition request input screen 92 of FIG. 12.

The candidate addition request input screen 92 includes, for each candidate compound, a chemical formula section 921 and an estimated physical property value section 922. The chemical formula section 921 indicates a chemical formula (chemical structural formula in FIG. 12) of the candidate compound, and the estimated physical property value section 922 indicates an estimated physical property value by the experimental design support device 10. The candidate addition request input screen 92 further includes a user feedback section 923.

In the user feedback section 923, the user inputs information on the availability of the candidate compound. In the example of FIG. 12, any one of "experimented", "postpone", or "unavailable" can be designated. In addition, in a case of experimented, the measurement value can be input. As described above, "experimented" and "postpone" indicate that the material is available (available material). "Experimented" indicates that measurement of the physical property value by experiment of the material has been conducted, and "postpone" indicates that no experiment has been conducted. "Unavailable" indicates that the material is unavailable to the user. When "experimented" is selected, the user also inputs the measurement value obtained by the experiment.

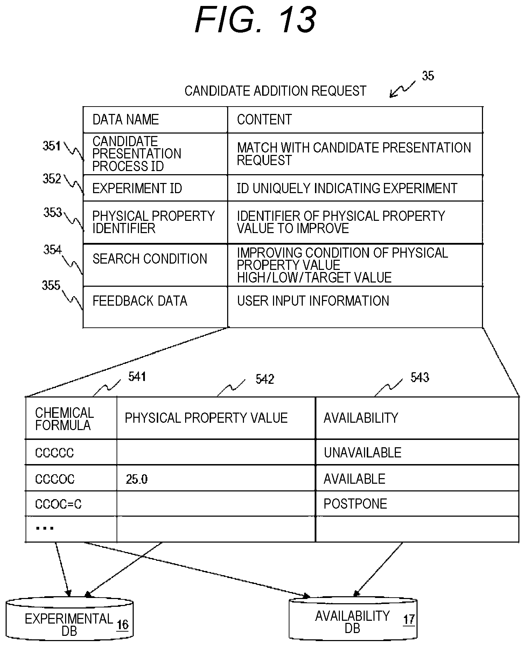

The candidate compound display unit 23 generates a candidate addition request 35 from the input user feedback information and information acquired from the experimental design support device 10 (displayed information transmission/reception unit 15) together with the candidate addition request input screen 92. FIG. 13 illustrates a configuration example of the candidate addition request 35. The candidate addition request 35 includes fields for a candidate presentation process ID 351, an experiment ID 352, a physical property identifier 353, a search condition 354, and feedback data 355.

The candidate presentation process ID 351 indicates an ID previously assigned to the experimental design support device 10. The experiment ID 352, the physical property identifier 353, and the search condition 354 are the same as the information of the field having the same name in the candidate presentation request 31 of the same project. For example, the candidate compound display unit 23 acquires these values from the displayed information transmission/reception unit 15.

The feedback data 355 indicates feedback information for each of the candidate compounds input by the user in the candidate addition request input screen 92. In the example of FIG. 13, each record of the feedback data 355 includes fields for a chemical formula 541, a physical property value 542, and availability 543. The chemical formula 541 represents a chemical formula of the compound, and the physical property value 542 represents a physical property value measured by an experiment. The availability 543 indicates a category for the availability of the compound, specifically, any of "experimented", "postpone", or "unavailable". Note that some data, for example, experimental data may not be included.

Returning to FIG. 11, in step S202, the displayed information transmission/reception unit 15 receives the candidate addition request 35 from the client computer 20. The displayed information transmission/reception unit 15 stores the experimental data indicated by the candidate addition request 35 in the experimental database 16, and stores information on the availability in the availability database 17. As a result, the information stored in the two databases 16 and 17 can be increased. The displayed information transmission/reception unit 15 stores the candidate addition request 35 in a database (not illustrated). The displayed information transmission/reception unit 15 further requests the candidate compound selection unit 13 for an additional candidate compound to present along with the candidate addition request 35.

Next, in step S203, the candidate compound generation unit 12 generates a candidate compound list. Specifically, the candidate compound selection unit 13 requests the candidate compound generation unit 12 to generate a candidate compound together with the candidate addition request 35 from which the feedback data 355 is eliminated. The candidate compound generation unit 12 generates a candidate compound list indicating the candidate compounds, and passes it to the candidate compound selection unit 13.

The candidate compound generation unit 12 can generate, for example, a new candidate compound from the compounds of the data of the project of the experimental database 16. In addition, a wider variety of candidate compounds may be generated by using data of compounds used in other applications. The candidate compound generation unit 12 may generate a new candidate compound from the available compounds of the project in the availability database 17. As the new candidate compound, an unavailable compound may be generated. The candidate compound generation unit 12 refers to the availability database 17 and excludes the already presented compound from the candidate compound list.

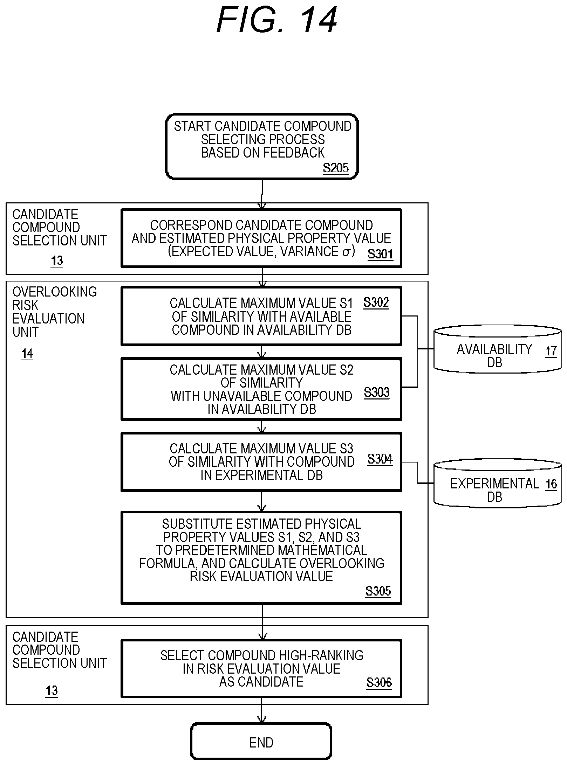

Next, in step S204, the physical property estimation unit 11 estimates the respective physical property values of the candidate compound list. Step S204 is similar to step 104 illustrated in FIG. 7. Next, in step S205, the candidate compound selection unit 13 executes a presentation candidate compound selecting process based on the user feedback. Details will be described later with reference to FIG. 14.

Next, in step S206, the displayed information transmission/reception unit 15 generates the candidate presentation screen 33 and transmits the screen to the client computer 20. The information on the candidate presentation screen 33 is similar to the information on the candidate presentation screen 33 transmitted in step S106 in FIG. 7 except that the compound is different. The displayed information transmission/reception unit 15 stores information of the selected candidate compound in a database (not illustrated) in association with the candidate addition request. In step S207, the candidate compound display unit 23 displays the candidate presentation screen 33 on the monitor 209.

FIG. 14 is a flowchart of an example of a candidate compound selecting process S205 based on user feedback. In FIG. 14, first, in step S301, the candidate compound selection unit 13 associates each candidate compound with the corresponding estimated physical property value (expected value and variance $\sigma$). The example described here estimates a probability distribution of the physical property values of the compound. As an example, the estimated physical property value is assumed to follow the Gaussian distribution. Such a probability distribution is obtained by, for example, a Gaussian process regression model.

Next, in response to the request from the candidate compound selection unit 13, the overlooking risk evaluation unit 14 executes steps S302 to S305 for each candidate compound. First, in step S302, the overlooking risk evaluation unit 14 calculates the similarity between each of the compounds that are available (experimented or postpone) in the data of the current project in the availability database 17 and the candidate compound. Any method can be used to calculate the similarity of the compounds. The overlooking risk evaluation unit 14 selects the maximum value S1 of the similarity.

Furthermore, in step S303, the overlooking risk evaluation unit 14 calculates similarity between each of the compounds unavailable in the data of the current project in the availability database 17 and the candidate compound. The overlooking risk evaluation unit 14 selects the maximum value S2 of the similarity.

Furthermore, in step S304, the overlooking risk evaluation unit 14 calculates similarity between each of the compounds in the data of the current project in the experimental database 16 and the candidate compound. The overlooking risk evaluation unit 14 selects the maximum value S3 of the similarity.

Next, in step S305, the overlooking risk evaluation unit 14 substitutes the estimated physical property values S1, S2, and S3 into a predetermined mathematical formula to calculate an overlooking risk evaluation value. The overlooking risk evaluation unit 14 returns the calculated overlooking risk evaluation values of all the candidate compounds to the candidate compound selection unit 13. In step S306, the candidate compound selection unit 13 selects a compound ranked high in the overlooking risk evaluation value as an experimental candidate to be presented.

An example of a method of calculating the overlooking risk evaluation value will be described. For example, the overlooking risk evaluation unit 14 can calculate the overlooking risk evaluation value f by the following mathematical formula (1). N represents a normal distribution.

[Mathematical formula 1]

$$f(X, S_1, S_2, S_3) = ln \int_{X_0}^{\infty} N(X - y; \sigma) dy + ln S_1 - ln S_2 - ln S_3 \qquad (1)$$

The above mathematical formula (1) can be used in an example in which the condition for improving the physical property value is maximized. In the above mathematical formula (1), X0 is a target value determined from the maximum value in the experimental data, and is, for example, the maximum value itself or a value smaller or larger than the maximum value by a predetermined value. The integral value indicates a probability that the physical property value of the candidate compound becomes greater than or equal to X0, and indicates an evaluation value (physical property evaluation value) for the physical property value of the candidate compound.

The terms of the similarities S1 and S2 indicate evaluation values for the availability of the candidate compound. A high similarity with an available compound means that a probability that a candidate compound is available is high. In addition, the high similarity with the unavailable compound means that a probability that the candidate compound is unavailable is high.

The overlooking risk evaluation value f increases as the probability that the physical property value becomes greater than or equal to X0 increases. In addition, it becomes larger the larger the similarity S1 with the available compound, and smaller the larger the similarity S2 with the unavailable compound. Furthermore, it becomes smaller the larger the similarity S3 to the experimented compound.

Based on the overlooking risk evaluation value f, a candidate compound estimated to have high probability of being available and exhibit good physical property values (with high probability) can be included in the candidate compound list to be presented to the user. In addition, even when the probability of being unavailable is high, the candidate compound estimated to exhibit extremely good physical property values can be included in the candidate compounds to be presented. Furthermore, it is possible to improve the efficiency of the experiment by excluding a compound having a high similarity with the experimented compound from the presentation list.

An overlooking risk evaluation value under other conditions of physical property values can be calculated by modifying the above mathematical formula (1). When the condition for improving the physical property value is minimized, for example, X0 in the above mathematical formula (1) is determined from the minimum value in the experimental data, and the range of integration is set to X0 to -∞, so that the overlooking risk evaluation value can be calculated. When the condition for improving the physical property value designates a target value, for example, the overlooking risk evaluation value can be calculated by setting the integration range to a predetermined range including the target value.

In the calculation of the evaluation value of the physical property value, a numerical value of the estimated physical property value may be used instead of the probability distribution of the physical property value. The integral in the above mathematical formula (1) is replaced with a function of the numerical value estimated by the physical property estimation unit 11. For example, the function of the condition of maximization becomes larger as the estimated value becomes larger, the function of the condition of minimization becomes larger as the estimated value becomes smaller, and the function of the condition for designating the target value becomes larger as it becomes closer to the target value.

The above mathematical formula (1) is an example, and the overlooking risk evaluation value may be calculated by other mathematical formulas. The above examples refer to the similarity with the experimented compound, but in other examples, reference to the similarity with the experimented compound may be omitted. The evaluation value of the availability may be calculated by a method different from the above method based on the similarities S1 and S2. For example, an evaluation value (e.g., SA score) of the synthesis difficulty determined from the chemical formula of the compound may be used, and one of S1 or S2 may be omitted. In both methods, the availability evaluation value is calculated based on the chemical formula of the candidate compound.

The overlooking risk evaluation unit 14 may calculate a plurality of overlooking risk evaluation values by different mathematical formulas (different criteria). The candidate compound selection unit 13 selects a high-ranking candidate compound from the overlooking risk evaluation value for each of the different mathematical formulas.

For example, mathematical formulas having different priorities (contributions) of physical property values, availability, and dissimilarities to the experimented compound can be prepared. A high priority means that a change in the value causes a greater change in the overlooking risk evaluation value.

For example, by increasing the weighting coefficient of the integral term of the above mathematical formula (1), the contribution of the term to the overlooking risk evaluation value increases, and the possibility of obtaining a compound exceeding the past physical property value can be prioritized. The presentation of the unavailable compound can be more reliably avoided by configuring the mathematical formula so as to increase the contribution of the term of the similarity with the unavailable compound, that is, so as to greatly decrease the evaluation value with respect to the increase in the similarity with the unavailable compound.

The candidate compound selection unit 13 may exclude one of the similar candidate compounds from the candidate compounds to present. For example, the candidate compound selection unit 13 may calculate the similarity between the compounds selected in accordance with the overlooking risk evaluation value, and exclude a compound having a low overlooking risk evaluation value from the compounds having a similarity exceeding a threshold value. A dissimilar compound having a lower overlooking risk evaluation value may be selected instead of the excluded compound. As a result, the range of compound candidates to be presented to the user can be expanded, and more efficient or development can be made.

Second Embodiment

Hereinafter, a second embodiment will be described. Differences from the first embodiment will be mainly described. In the second embodiment, a history of presentation of candidate compounds is used in the calculation of the overlooking risk evaluation value. In the second embodiment, as the number of presentations of the candidate compound increases, the possibility that a candidate compound that has not been selected before will be presented increases. Thus, the number of experiments can be avoided from continuously increasing without specific candidate compounds being presented. This makes it possible to present, for example, a compound having low availability but exhibiting satisfactory physical property values, and to reduce the possibility that a desired compound cannot be obtained even if the experiment is repeated.

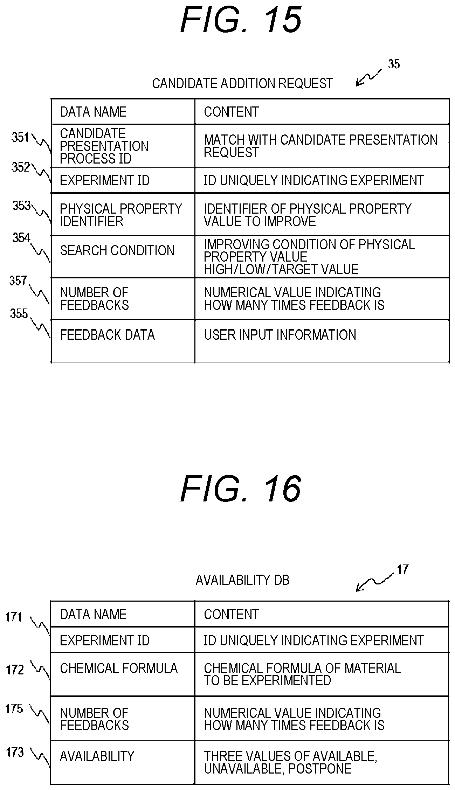

FIG. 15 illustrates a configuration example of a candidate addition request 35 according to a second embodiment. A field for the number of feedbacks 357 is added to the candidate addition request according to the first embodiment illustrated in FIG. 13. In this way, the present embodiment manages the number of feedbacks in the project, that is, the number of presentations of the candidate compound or the number of experiments.

For example, the displayed information transmission/reception unit 15 of the experimental design support device 10 manages a series of candidate addition requests, and includes information on the number of feedbacks in the data of the candidate addition request input screen 92 to be transmitted to the client computer 20. The candidate compound display unit 23 of the client computer 20 stores the numerical value determined with reference to the number of times in the field for the number of feedbacks 357.

FIG. 16 illustrates a configuration example of an availability database 17 according to the second embodiment. A field for the number of feedbacks 175 is added to the availability database according to the first embodiment illustrated in FIG. 4. The displayed information transmission/reception unit 15 stores the number of feedbacks indicated by the received candidate addition request 35 in the field for the number of feedbacks 175 together with other data.

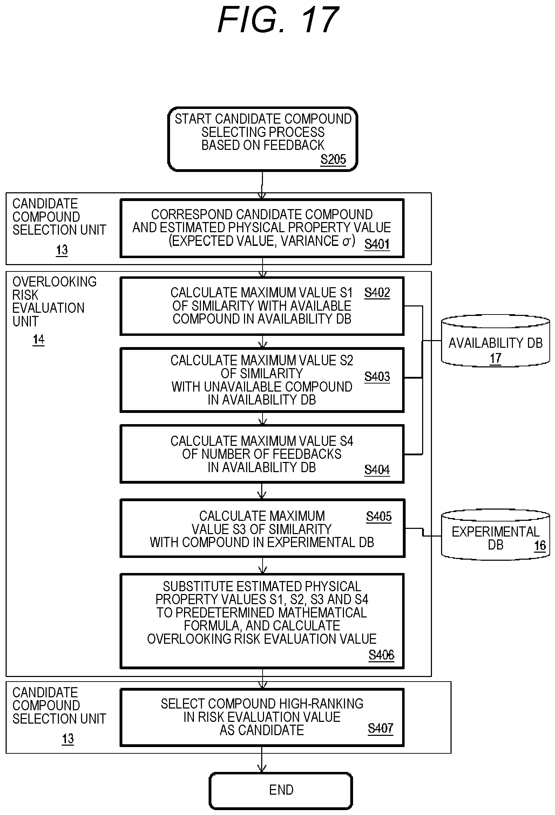

FIG. 17 is a flowchart of an example of a candidate compound selecting process S205 based on user feedback according to the second embodiment. Steps S401 to S403 are similar to steps S301 to S303 of the flowchart illustrated in FIG. 14. In step S404, the overlooking risk evaluation unit 14 calculates the maximum value S4 of the number of feedbacks of the project in the availability database 17. Step S405 is similar to step S304 of the flowchart illustrated in FIG. 14.

Next, in step S406, the overlooking risk evaluation unit 14 substitutes the estimated physical property values S1, S2, S3 and S4 into a predetermined mathematical formula to calculate an overlooking risk evaluation value. The overlooking risk evaluation unit 14 returns the calculated overlooking risk evaluation values of all the candidate compounds to the candidate compound selection unit 13. In step S407, the candidate compound selection unit 13 selects a high-ranking compound in the overlooking risk evaluation value as an experimental candidate.

An example of a method of calculating the overlooking risk evaluation value will be described. For example, the overlooking risk evaluation unit 14 can calculate the overlooking risk evaluation value f by the following mathematical formula (2).

[Mathematical formula 2]

$$f(X, S_1, S_2, S_3, S_4) = \ln \int_{X_0}^{\infty} N(X - y; \sigma) dy + \ln S_1 - \ln S_2 - \ln S_3 + S_4 rand() \qquad (2)$$

In the above mathematical formula (2), a random number element S4rand () is added to the mathematical formula (1) in the first embodiment. rand() means a random number. The number of feedbacks S4 increases with the number of experiments. Therefore, as the number of experiments (the number of presentations or the number of feedbacks) increases, the magnitude of the random number element S4rand() increases, and the contribution to the overlooking risk evaluation value f increases.

That is, regardless of the physical property value, the availability, and the similarity to the experimented compound, the overlooking risk evaluation value f of the randomly selected candidate compound increases. As a result, a compound under a condition that has not been selected so far is selected as a compound to be presented, and as the number of experiments increases, the possibility can be increased.

The overlooking risk evaluation unit 14 can calculate the overlooking risk evaluation value f by the following mathematical formula (3) in place of or in addition to the above mathematical formula (2).

[Mathematical formula 3]

$$f(X, S_1, S_2, S_3, S_4) = \ln \int_{X_0}^{\infty} N(X - y; \sigma) dy + (\ln S_1 - \ln S_2 - \ln S_3)/S_4 \qquad (3)$$

In the above mathematical formula (3), a divisor S4 for (lnS1 - lnS2 - lnS3) is added to the mathematical formula (1) in the first embodiment. The number of feedbacks S4 increases with the number of experiments. Therefore, as the number of experiments (the number of presentations) increases, the contribution of the evaluation value of an element other than the physical property value to the overlooking risk evaluation value f decreases. The priority of the physical property value in the overlooking risk evaluation value f is relatively high. That is, the possibility that a candidate compound exhibiting good physical property values is selected increases regardless of the availability and the similarity with the experimented compound. As a result, compounds that have not been selected due to factors other than physical property values can be included in the presentation list.

The candidate compound selection unit 13 may use, for example, one of mathematical formula (2) or mathematical formula (3) to select a candidate compound to present. A predetermined number of candidate compounds having a high overlooking risk evaluation value f are selected. In another example, the candidate compound selection unit 13 may select a predetermined number of candidate compounds high-ranking in the overlooking risk evaluation values f of each of the mathematical formulas (2) and (3). A more exhaustive candidate compounds can be selected by using a plurality of mathematical formulas.

Note that the present invention is not limited to the embodiments described above, and includes various modified examples. For example, the embodiments described above have been described in detail for the sake of easy understanding of the present invention, and are not necessarily limited to those having all the described configurations. In addition, a part of the configuration of a certain embodiment can be replaced with a configuration of another embodiment, and the configuration of a certain embodiment can be added with the configuration of another embodiment. Furthermore, for a part of the configuration of each embodiment, other configurations can be added, deleted, and replaced.

In addition, some or all of the above-described configurations, functions, processing units, and the like may be realized by hardware, for example, by designing with an integrated circuit. In addition, each of the above-described configurations, functions, and the like may be realized by software by a processor interpreting and executing a program for realizing each function. Information such as a program, a table, and a file for realizing each function can be stored in a recording device such as a memory, a hard disk, and a solid state drive (SSD), or a recording medium such as an IC card and an SD card.

In addition, control lines and information lines that are considered necessary for the description are shown, and not all control lines and information lines are necessarily shown in terms of product. In practice, it may be considered that almost all the configurations are connected to each other.

**Claims**

1. A system for determining a material to propose to a user, the system comprising:

   one or more processors; and
   one or more storage devices that store programs to be executed by the one or more processors,
   wherein
   the one or more processors
   calculate an availability evaluation value indicating availability for a user of each of a plurality of materials based on a chemical formula of each of the plurality of materials,
   estimate a physical property value of each of the plurality of materials based on a chemical formula of each of the plurality of materials,
   calculate a physical property evaluation value of each of the plurality of materials based on an estimation result of the physical property value of each of the plurality of materials,
   calculate an overlooking risk evaluation value indicating priority of presenting each of the plurality of materials to the user based on the availability evaluation value and the physical property evaluation value of each of the plurality of materials, and
   select a material to present as a candidate material from the plurality of materials according to the overlooking risk evaluation value.

2. The system according to claim 1, wherein

   the one or more storage devices store a first database that stores information on a material for which a physical property value has been measured, and
   the one or more processors calculate the overlooking risk evaluation value further based on similarity between each of the plurality of materials and a chemical formula of the material for which the physical property value has been measured.

3. The system according to claim 1, wherein the physical property evaluation value is based on a probability that a physical property value of each of the plurality of materials becomes greater than or equal to a target value.

4. The system according to claim 1, wherein

   the one or more storage devices store a second database that stores information on an available material and an unavailable material, and
   the one or more processors calculate an availability evaluation value of each of the plurality of materials based on similarity between a chemical formula of the available material and the unavailable material and a chemical formula of each of the plurality of materials.

5. The system according to claim 4, wherein

   the one or more processors
   receive feedback information from a user for a presented candidate material, the feedback information including information on availability for at least a part of the presented candidate material, and
   stores the information on availability in the feedback information in the second database.

6. The system according to claim 5, wherein

   the one or more storage devices store a first database that stores information on a material for which a physical property value has been measured,
   the one or more processors calculate the overlooking risk evaluation value further based on similarity between each of the plurality of materials and a chemical formula of the material for which the physical property value has been measured,
   the feedback information includes a measured physical property value of at least a part of the presented candidate material, and
   the one or more processors store the measured physical property value in the feedback information in the first database.

7. The system according to claim 1, wherein in calculation of the overlooking risk evaluation value, a priority of the physical property evaluation value increases according to number of presentations of the candidate material.

8. The system according to claim 1, wherein the calculation of the overlooking risk evaluation value is based on a random number element that increases according to number of presentations of the candidate material.

9. The system according to claim 1, wherein

the one or more processors
calculate a plurality of overlooking risk evaluation values by different mathematical formulas for each of the plurality of materials, and
select a material to present as a candidate material from the plurality of materials based on the plurality of overlooking risk evaluation values.

10. The system according to claim 1, wherein the one or more processors exclude one of the materials, in which the similarity of the chemical formula exceeds a threshold value, from the candidate materials to present.

11. A method for determining a material to propose to a user, the system including one or more processors, and one or more storage devices that store programs to be executed by the one or more processors, the method comprising the steps of:

the one or more processors calculating an availability evaluation value indicating availability for a user of each of a plurality of materials based on a chemical formula of each of the plurality of materials;
the one or more processors estimating a physical property value of each of the plurality of materials based on a chemical formula of each of the plurality of materials;
the one or more processors calculating a physical property evaluation value of each of the plurality of materials based on an estimation result of the physical property value of each of the plurality of materials;
the one or more processors calculating an overlooking risk evaluation value indicating priority of presenting each of the plurality of materials to the user based on the availability evaluation value and the physical property evaluation value of each of the plurality of materials; and
the one or more processors selecting a material to present as a candidate material from the plurality of materials according to the overlooking risk evaluation value.

# FIG. 1

**EXPERIMENTAL DESIGN SUPPORT DEVICE** 10

| PHYSICAL PROPERTY ESTIMATION UNIT 11 | CANDIDATE COMPOUND GENERATION UNIT 12 |

| CANDIDATE COMPOUND SELECTION UNIT 13 | OVERLOOKING RISK EVALUATION UNIT 14 |

AVAILABILITY DB 17

| DISPLAYED INFORMATION TRANSMISSION/ RECEPTION UNIT 15 |

EXPERIMENTAL DB 16

**CLIENT COMPUTER** 20

**Web BROWSER** 21

CANDIDATE COMPOUND DISPLAY UNIT 23

15

# FIG. 2

EXPERIMENTAL DESIGN SUPPORT DEVICE    10

| PROCESSOR 205 | AUXILIARY STORAGE DEVICE 207 | MONITOR 209 |
| DRAM 208 | INPUT DEVICE    210 | COMMUNICATION DEVICE 206 |

CLIENT COMPUTER    20

| PROCESSOR 205 | AUXILIARY STORAGE DEVICE 207 | MONITOR 209 |
| DRAM 208 | INPUT DEVICE    210 | COMMUNICATION DEVICE 206 |

# FIG. 3

EXPERIMENTAL DB

16

| DATA NAME | CONTENT |
|---|---|
| EXPERIMENT ID | ID UNIQUELY INDICATING EXPERIMENT |
| CHEMICAL FORMULA | CHEMICAL FORMULA OF MATERIAL TO BE EXPERIMENTED |
| PHYSICAL PROPERTY IDENTIFIER | TYPE NAME OF MEASURED PHYSICAL PROPERTY VALUE |
| PHYSICAL PROPERTY VALUE | MEASURED PHYSICAL PROPERTY VALUE |

# FIG. 4

AVAILABILITY DB

17

| DATA NAME | CONTENT |
|---|---|
| EXPERIMENT ID | ID UNIQUELY INDICATING EXPERIMENT |
| CHEMICAL FORMULA | CHEMICAL FORMULA OF MATERIAL |
| AVAILABILITY | THREE VALUES OF EXPERIMENTED, UNAVAILABLE, POSTPONE |

# FIG. 5

EP 4 145 452 A1

# FIG. 6

# FIG. 7

```
┌─────────────────────────────────────────────┐
│   START INITIAL EXPERIMENT CANDIDATE          │
│     COMPOUND PRESENTING PROCESS               │
└─────────────────────────────────────────────┘
                      │
                      ▼
CANDIDATE          ┌─────────────────────────────────┐
COMPOUND           │   TRANSMIT CANDIDATE             │
DISPLAY UNIT       │   PRESENTATION REQUEST    S101    │
23                 └─────────────────────────────────┘
                      │
                      ▼
DISPLAYED          ┌─────────────────────────────────┐
INFORMATION        │ RECEIVE CANDIDATE PRESENTATION   │
TRANSMISSION/      │ REQUEST, AND REQUEST             │
RECEPTION UNIT     │ CORRESPONDING CANDIDATE   S102    │
15                 │ COMPOUND                         │
                   └─────────────────────────────────┘
                      │
                      ▼
CANDIDATE          ┌─────────────────────────────────┐
COMPOUND           │ GENERATE CANDIDATE COMPOUND LIST │
GENERATION         │ (REINFORCEMENT LEARNING MODEL,   │
UNIT               │ VAE, GAN, ETC.)           S103    │
12                 └─────────────────────────────────┘
                      │
                      ▼
PHYSICAL           ┌─────────────────────────────────┐
PROPERTY           │ ESTIMATE RESPECTIVE PHYSICAL     │
ESTIMATION UNIT    │ PROPERTY VALUE OF CANDIDATE      │
11                 │ COMPOUND LIST             S104    │
                   └─────────────────────────────────┘
                      │
                      ▼
CANDIDATE          ┌─────────────────────────────────┐
COMPOUND           │ SELECT COMPOUND WHOSE ESTIMATED  │
SELECTION UNIT     │ PHYSICAL PROPERTY VALUE IS       │
13                 │ CLOSE TO IDEAL VALUE      S105    │
                   │ DEFINED IN CANDIDATE             │
                   │ PRESENTATION REQUEST             │
                   └─────────────────────────────────┘
                      │
                      ▼
DISPLAYED          ┌─────────────────────────────────┐
INFORMATION        │ GENERATE CANDIDATE               │
TRANSMISSION/      │ PRESENTATION SCREEN AND          │
RECEPTION UNIT     │ TRANSMIT TO CLIENT        S106    │
15                 │ COMPUTER                         │
                   └─────────────────────────────────┘
                      │
                      ▼
CANDIDATE          ┌─────────────────────────────────┐
COMPOUND           │ DISPLAY CANDIDATE                │
DISPLAY UNIT       │ PRESENTATION SCREEN       S107    │
23                 └─────────────────────────────────┘
                      │
                      ▼
              ┌──────────────┐
              │     END      │
              └──────────────┘
```

# FIG. 8

91

-C-COMPOUND CANDIDATE PRESENTATION SERVICE - Web Viewer

← → FILE(F)　　EDIT(E)　　SETTING(S)　　HELP(H)

URL: http://www.material.org/view3d/main.html

CANDIDATE COMPOUND GENERATION FUNCTION CALLOUT SCREEN

EXPERIMENT NAME　　　PERFORMANCE IMPROVEMENT PROJECT OF ORGANIC XX ▼　911

PHYSICAL PROPERTY VALUE TYPE　　ELECTRICAL CONDUCTIVITY ▼　912

913
CONDITION　　○ MAXIMUM
　　　　　　● MINIMUM
　　　　　　○ TARGET VALUE

EXECUTE　　CANCEL

COMMUNICATION COMPLETE

# FIG. 9

31

| DATA NAME | CONTENT |
|---|---|
| CANDIDATE PRESENTATION PROCESS ID | BLANK IN INITIAL STATE |
| EXPERIMENT ID | ID UNIQUELY INDICATING EXPERIMENT |
| PHYSICAL PROPERTY IDENTIFIER | IDENTIFIER OF PHYSICAL PROPERTY VALUE TYPE TO IMPROVE |
| SEARCH CONDITION | IMPROVING CONDITION OF PHYSICAL PROPERTY VALUE HIGH/LOW/TARGET VALUE |

311
312
313
314

CANDIDATE PRESENTATION REQUEST

# FIG. 10

GENERATE
CHEMICAL FORMULA

ASSIGN
ESTIMATED
VALUE

CANDIDATE
COMPOUND
GENERATION
UNIT
12

CHEMICAL
FORMULA A

1.3

CHEMICAL
FORMULA B

2.1

CHEMICAL
FORMULA C

0.8

CHEMICAL
FORMULA D

2.8

PHYSICAL
PROPERTY
ESTIMATION
UNIT
11

CANDIDATE COMPOUND
SELECTION UNIT    13

LEAVE
HIGH-RANKING X
OF ESTIMATED
VALUE

CHEMICAL
FORMULA D

2.8

CHEMICAL
FORMULA B

2.1

# FIG. 11

START EXPERIMENTAL CANDIDATE COMPOUND
ADDITION PRESENTING PROCESS

| CANDIDATE COMPOUND DISPLAY UNIT 23 | TRANSMIT CANDIDATE ADDITION REQUEST INCLUDING FEEDBACK INFORMATION S201 |

| DISPLAYED INFORMATION TRANSMISSION/ RECEPTION UNIT 15 | RECEIVE CANDIDATE ADDITION REQUEST AND RECORD IN DB, REQUEST CANDIDATE COMPOUND SELECTION UNIT FOR CORRESPONDING CANDIDATE COMPOUND S202 |

EXPERIMENTAL DB 16

AVAILABILITY DB 17

| CANDIDATE COMPOUND GENERATION UNIT 12 | GENERATE CANDIDATE COMPOUND LIST (REINFORCEMENT LEARNING MODEL, VAE, GAN, ETC.) S203 |

| PHYSICAL PROPERTY ESTIMATION UNIT 11 | ESTIMATE RESPECTIVE PHYSICAL PROPERTY VALUES OF CANDIDATE COMPOUND LIST S204 |

| CANDIDATE COMPOUND SELECTION UNIT 13 | CANDIDATE COMPOUND SELECTING PROCESS BASED ON FEEDBACK S205 |

OVERLOOKING RISK EVALUATION UNIT 14

| DISPLAYED INFORMATION TRANSMISSION/ RECEPTION UNIT 15 | GENERATE CANDIDATE PRESENTATION SCREEN, AND TRANSMIT TO CLIENT COMPUTER S206 |

| CANDIDATE COMPOUND DISPLAY UNIT 23 | DISPLAY CANDIDATE PRESENTATION SCREEN S207 |

END

# FIG. 12

92

-C-COMPOUND CANDIDATE PRESENTATION SERVICE - Web Viewer ☐ ◼ X

← → FILE(F)    EDIT(E)    SETTING(S)    HELP(H)

URL: http://www.material.org/view3d/main.html

LIST OF CANDIDATE COMPOUNDS      CANDIDATE PRESENTATION
                                  PROCESS ID=254

COMPOUND 1          923
                              MEASUREMENT        921        922
                                 VALUE
   ○ EXPERIMENTED                            C                ESTIMATED
                                                              PHYSICAL
   ● POSTPONE                         C        C              PROPERTY
                                 C                            VALUE
   ○ UNAVAILABLE                                   NH         22.5

COMPOUND 2

                                              EXECUTE      CANCEL

COMMUNICATION COMPLETE

# FIG. 13

CANDIDATE ADDITION REQUEST ~ 35

| DATA NAME | CONTENT |
|---|---|
| 351 CANDIDATE PRESENTATION PROCESS ID | MATCH WITH CANDIDATE PRESENTATION REQUEST |
| 352 EXPERIMENT ID | ID UNIQUELY INDICATING EXPERIMENT |
| 353 PHYSICAL PROPERTY IDENTIFIER | IDENTIFIER OF PHYSICAL PROPERTY VALUE TO IMPROVE |
| 354 SEARCH CONDITION | IMPROVING CONDITION OF PHYSICAL PROPERTY VALUE HIGH/LOW/TARGET VALUE |
| 355 FEEDBACK DATA | USER INPUT INFORMATION |

| CHEMICAL FORMULA | PHYSICAL PROPERTY VALUE ~ 542 | AVAILABILITY ~ 543 |
|---|---|---|
| CCCCC | | UNAVAILABLE |
| CCCOC | 25.0 | AVAILABLE |
| CCOC=C | | POSTPONE |
| ... | | |

~ 541

EXPERIMENTAL DB 16

AVAILABILITY DB 17

# FIG. 14

```
┌──────────────────────────────┐
│  START CANDIDATE COMPOUND    │
│     SELECTING PROCESS        │
│     BASED ON FEEDBACK        │
│                      S205    │
└──────────────────────────────┘
```

CANDIDATE
COMPOUND
SELECTION UNIT
13

CORRESPOND CANDIDATE COMPOUND
AND ESTIMATED PHYSICAL PROPERTY VALUE
(EXPECTED VALUE, VARIANCE $\sigma$)   S301

OVERLOOKING
RISK
EVALUATION
UNIT
14

CALCULATE MAXIMUM VALUE S1   S302
OF SIMILARITY WITH AVAILABLE
COMPOUND IN AVAILABILITY DB

CALCULATE MAXIMUM VALUE S2
OF SIMILARITY
WITH UNAVAILABLE COMPOUND   S303
IN AVAILABILITY DB

AVAILABILITY   17
DB

CALCULATE MAXIMUM VALUE S3
OF SIMILARITY WITH COMPOUND   S304
IN EXPERIMENTAL DB

EXPERIMENTAL   16
DB

SUBSTITUTE ESTIMATED PHYSICAL
PROPERTY VALUES S1, S2, AND S3
TO PREDETERMINED MATHEMATICAL
FORMULA, AND CALCULATE OVERLOOKING
RISK EVALUATION VALUE
S305

CANDIDATE
COMPOUND
SELECTION UNIT
13

SELECT COMPOUND HIGH-RANKING
IN RISK EVALUATION VALUE
AS CANDIDATE   S306

END

# FIG. 15

CANDIDATE ADDITION REQUEST ⟋ 35

| DATA NAME | CONTENT |
|---|---|
| CANDIDATE PRESENTATION PROCESS ID | MATCH WITH CANDIDATE PRESENTATION REQUEST |
| EXPERIMENT ID | ID UNIQUELY INDICATING EXPERIMENT |
| PHYSICAL PROPERTY IDENTIFIER | IDENTIFIER OF PHYSICAL PROPERTY VALUE TO IMPROVE |
| SEARCH CONDITION | IMPROVING CONDITION OF PHYSICAL PROPERTY VALUE HIGH/LOW/TARGET VALUE |
| NUMBER OF FEEDBACKS | NUMERICAL VALUE INDICATING HOW MANY TIMES FEEDBACK IS |
| FEEDBACK DATA | USER INPUT INFORMATION |

# FIG. 16

AVAILABILITY DB ⟋ 17

| DATA NAME | CONTENT |
|---|---|
| EXPERIMENT ID | ID UNIQUELY INDICATING EXPERIMENT |
| CHEMICAL FORMULA | CHEMICAL FORMULA OF MATERIAL TO BE EXPERIMENTED |
| NUMBER OF FEEDBACKS | NUMERICAL VALUE INDICATING HOW MANY TIMES FEEDBACK IS |
| AVAILABILITY | THREE VALUES OF AVAILABLE, UNAVAILABLE, POSTPONE |

# FIG. 17

```
              ┌─────────────────────────────┐
              │ START CANDIDATE COMPOUND    │
              │ SELECTING PROCESS           │
              │ BASED ON FEEDBACK           │
              │                    S205     │
              └─────────────────────────────┘
```

**CANDIDATE COMPOUND SELECTION UNIT** 13

CORRESPOND CANDIDATE COMPOUND AND ESTIMATED PHYSICAL PROPERTY VALUE (EXPECTED VALUE, VARIANCE $\sigma$)  S401

**OVERLOOKING RISK EVALUATION UNIT** 14

CALCULATE MAXIMUM VALUE S1 OF SIMILARITY WITH AVAILABLE COMPOUND IN AVAILABILITY DB  S402

CALCULATE MAXIMUM VALUE S2 OF SIMILARITY WITH UNAVAILABLE COMPOUND IN AVAILABILITY DB  S403

CALCULATE MAXIMUM VALUE S4 OF NUMBER OF FEEDBACKS IN AVAILABILITY DB  S404

**AVAILABILITY DB** 17

CALCULATE MAXIMUM VALUE S3 OF SIMILARITY WITH COMPOUND IN EXPERIMENTAL DB  S405

**EXPERIMENTAL DB** 16

SUBSTITUTE ESTIMATED PHYSICAL PROPERTY VALUES S1, S2, S3 AND S4 TO PREDETERMINED MATHEMATICAL FORMULA, AND CALCULATE OVERLOOKING RISK EVALUATION VALUE  S406

**CANDIDATE COMPOUND SELECTION UNIT** 13

SELECT COMPOUND HIGH-RANKING IN RISK EVALUATION VALUE AS CANDIDATE  S407

END

**INTERNATIONAL SEARCH REPORT**

| International application No. |
| --- |
| PCT/JP2021/015045 |

**A. CLASSIFICATION OF SUBJECT MATTER**
Int.Cl. G16C20/40(2019.01)i
FI: G16C20/40

According to International Patent Classification (IPC) or to both national classification and IPC

**B. FIELDS SEARCHED**

Minimum documentation searched (classification system followed by classification symbols)
Int.Cl. G16C20/40

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2020-30638 A (PANASONIC INTELLECTUAL PROPERTY MANAGEMENT CO., LTD.) 27 February 2020 (2020-02-27), entire text, all drawings | 1-11 |
| A | JP 2004-500614 A (NOVASCREEN BIOSCIENCES CORPORATION) 18 January 2004 (2004-01-18), entire text, all drawings | 1-11 |
| A | WO 2020/054839 A1 (FUJIFILM CORPORATION) 19 March 2020 (2020-03-19), entire text, all drawings | 1-11 |

☐ Further documents are listed in the continuation of Box C.    ☒ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 14 June 2021 | 22 June 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

| | | | International application No. |
|---|---|---|---|
| **INTERNATIONAL SEARCH REPORT** Information on patent family members | | | PCT/JP2021/015045 |

| | | |
|---|---|---|
| JP 2020-30638 A | 27 February 2020 | (Family: none) |
| JP 2004-500614 A | 18 January 2004 | US 2002/0187514 A1 the whole document WO 2000/065421 A2 AU 4661300 A CA 2371093 A1 |
| WO 2020/054839 A1 | 19 March 2020 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020080755 A

- JP 2014092930 A